# EUROPEAN PATENT APPLICATION

(11) **EP 1 661 522 A1**
(43) Date of publication of application: **31.05.2006**
(21) Application number: 04771510.7
(22) Date of filing: 04.08.2004
(51) Int. Cl.: A61B 17/56, A61B 18/04

(54) **DISC HERNIATION TREATING METHOD AND APPARATUS**

(30) Priority: 07.08.2003 JP 2003288572
(71) Applicant: Teijin Pharma Limited, Tokyo 100-0011 (JP)
(72) Inventor: IWABUCHI, Sadahiro, c/o Teijin Pharma Limited, Hino-shi, Tokyo 1910065 (JP); ITO, Masaya, c/o Teijin Limited, Hino-shi, Tokyo 1910065 (JP); AZUMA, Yoshiaki, c/o Teijin Pharma Limited, Hino-shi, Tokyo 1910065 (JP)
(74) Representative: HOFFMANN EITLE
(86) International application number: PCT/JP2004/011525
(87) International publication number: WO 2005/013838

(57) **Abstract**

As a method of promoting natural HDR and curing disc herniation an apparatus is provided for shortening the time period of HDR, by ultrasonic irradiation. The apparatus is equipped with at least one ultrasonic transducer, an ultrasonic oscillator and mount means for mounting the ultrasonic transducer to an bone joint site, and is means for irradiating ultrasonic waves of 1.3 to 2MHz in frequency, 100 to 1,000Hz in repetition frequency, 10 to 2000µs in burst width and 100mW/cm² or less in power (SATA: Spatial Average-Temporal Average).

## Description

### Technical Field

The present invention relates to a natural resorption promoting device and method for herniated disc (HD). More particularly, the present invention relates to an apparatus for spurring new blood vessel growth by infiltration of macrophages (Mφs) in ruptured position and increase the activity of matrix-metalloprotease (MMP) to promote HD repairing inflammatory reaction, thereby shortening the time period of natural HD resorption (HDR).

### Background Art

HD is a disorder caused by bulge, protrusion of the nucleus pulposus (NP) and annulus fibrosus (AF) in the intervertebral disc (ID), and also it is a disorder causing symptoms such as extremity and lumbar pains, etc. appearing because the prolapsed site stimulates surrounding nerves. HD is classified into the following four kinds.
1) a bulge type of HD in which surrounding nerves are simulated by the bulge of NP and AF, so that symptoms such as pain appear.
2) A protrusion type of HD in which the NP and AF in the ID protrude into the vertebral canal after bulging; however, they do not penetrate and break the posterior longitudinal ligament.
3) A prolapsed type of HD in which NP and AF are further prolapsed into the vertebral canal, and penetrate and break the posterior longitudinal ligament, but however are not detached from the ID.
4) A detached type in which the NP and AF are prolapsed into the vertebral canal, and then detached from the ID.

For example, it has been reported that the population of people having HD is higher in men than that in women, the age of most frequent occurrence is from the 20's to 40's in both men and women, and 40% of patients having back pain is caused by HD of lumbar vertebra. The back pain is the top chief complaint in out-patient clinic of the orthopedic surgery, and the total number of patients having disc disorder is equal to 347, 000 persons (investigated in 1996 by Health and Welfare Ministry, and it is on the increase. 70% of present medical treatment for HD comprises conservative treatments such as complete rest or wet compress for desensitization, administration of anti-inflammatory painkilling drug, physical therapies such as traction, thermal therapy, and gymnastic exercises, and nerve block treatments such as caudal epidural block, nerve root block. In addition, surgical methods such as ruptured NP ablation, percutaneous laser ablation are conducted, for patients resistant to conservative treatments or for the purpose of early return to normal life.

The following problems with the present conservative treatments have been noted.
(1) The medical curing time is very long, from three months to one year or more.
(2) When lessening of HD by the conservative treatments is not perceived, an invasive surgical medical treatment must be carried out.
(3) There are many cases where some patients mistakenly judge that the HD is perfectly cured because pain is gone, and thus hernia occurs again.

It has been recently found from analysis results of magnetic resonance image (MRI) that HD is cured naturally. From clinical symptoms, etc., it has been found that the features of the natural HDR are as follows:
1) HDR is more likely to occur as blood circulation is better, 2) Infiltration of T lymph corpuscles containing Mφs into prolapsed herniated portions is increased,
3)Existence of granulation tissue indicating increasing growth of new blood vessel is confirmed,
4) Activity of MMP-3 (stromelysin-1) is increased.
For basic research clarifying the mechanism of HDR, using an in -vitro HDR mouse model in which co-existing culture of the mouse ID and penetrated Mφ, reduction of wet weight of the ID, increase the release of MMP-3, etc. have been observed (Hirotaka Haro, et al., The Journal of Clinical Investigation, Vol. 105, 2000). Recently, it has been recommended that HD patients excluding urination trouble should be treated the conservative therapy for at least three months. So, a non-invasive new conservative therapy that has a short duration and can be expected to have a therapeutic effect has been desired.

### Disclosure of the Invention

It is theorized that by exposing herniated tissue to blood circulation, increasing growth of new blood vessel and infiltration of Mφ may occur and thus degeneration of tissue by MMP may be carried out. An object of the present invention is to provide a new medical treatment method for HD on the basis of the above theory. In order to attain the above object, there is provided a method and apparatus that shorten the duration of HDR, avoiding surgery such as ablation of HD.

The inventors have been dedicated to studies on a method of enhancing the activity of new blood vessel growth based on Mφ and increase of the MMP activity in connection with the disorder of HD, and have found the following apparatus and method.

That is, there is provided an apparatus for promoting the resorption process of HD by irradiation with ultrasound, equipped with at least one ultrasonic transducer, an ultrasonic oscillator and mount means for mounting the ultrasonic transducer to the site of HD. Furthermore, according to the present invention, there is provided an apparatus for promoting the HDR comprising an ultrasonic transducer as means for emitting ultrasound having a frequency of 1.3 to 2MHz, a repetition frequency of 100 to 1,000Hz, a burst width of 10 to 2000µs and power of 1 to 100mW/cm² (SATA: Spatial Average-Temporal Average), and particularly, the ultrasound have frequency of 1.5MHz, repetition frequency of 1kHz, burst width of 200µs and power of 30mW/cm².

Furthermore, according to the present invention, there is provided a method of promoting HDR in which ultrasound is emitted from above the surface of skin to the site of HD. Furthermore, there is provided a method of promoting HDR in which the ultrasonic waves are set to have a frequency of 1.3 to 2MHz, a repetition frequency of 100 to 1,000Hz, a burst width of 10 to 2000µs and power of 1 to 100mw/cm² (SATA: Spatial Average-Temporal Average), and particularly, the ultrasonic waves are set to have a frequency of 1.5MHz, a repetition frequency of 1kHz, a burst width of 200µs and power of 30mW/cm².

Furthermore, there is provided a medical treatment method for HD in which ultrasound is irradiated from above the surface of skin to the site of HD. Particularly, there is provided a medical treatment method for HD in which the ultrasound is set to have a frequency of 1.3 to 2MHz, a repetition frequency of 100 to 1,000Hz, a burst width of 10 to 2000µs, and power of 1 to 100mW/cm² (SATA: Spatial Average-Temporal Average), and more particularly the ultrasound is set to have a frequency of 1.5MHz, a repetition frequency of 1kHz, a burst width of 200µs and power of 30mW/cm².

### Brief Description of the Drawings

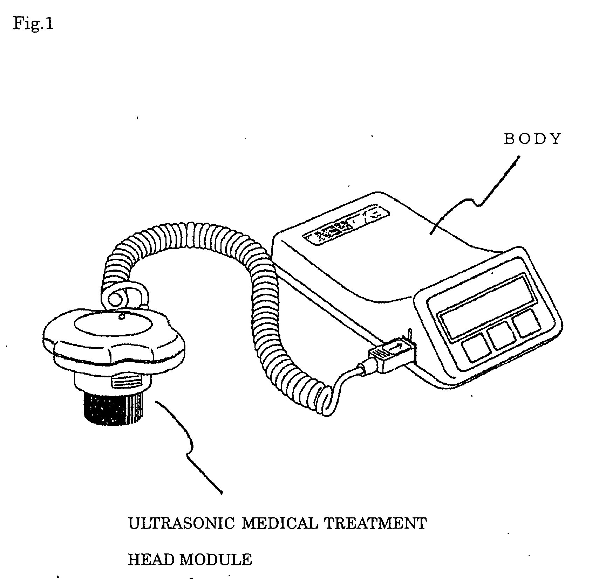

Fig. 1 is an external view of an apparatus for promoting HDR according to the present invention.

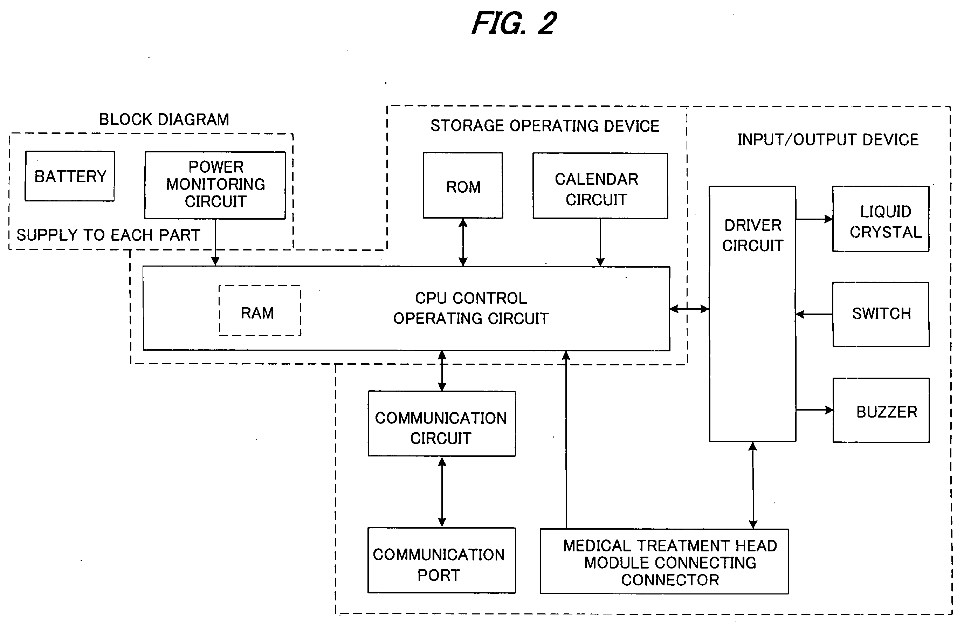

Fig. 2 is a block diagram showing the main body of the apparatus for promoting HDR according to the present invention.
Fig. 3 is a block diagram showing an ultrasonic head module of the main body of the apparatus for promoting HDR according to the present invention.
Fig. 4 is an external view and a frame outline diagram of an ultrasonic irradiation device for experiments.
Fig. 5 is a diagram showing variation of the wet weight of the ID.
Fig. 6 is a diagram showing an osteopontin concentration measured by an ELISA method.
Fig. 7 is a diagram showing MCP-1 concentration measured by the ELISA method.
Fig. 8 is an HE-dyed image.
Fig. 9 shows an MMP-3 dyed image. An arrow shows a positive reaction.
Fig. 10 is a diagram showing the overall MMP-3 immunostaining results in extracellular matrix.
Fig. 11 is a diagram showing the overall apoptosis dyeing results.

### Best Modes for Carrying out the Invention

The present invention will be described hereunder in detail.

### [Apparatus for promoting natural resorption of herniated disc]

An apparatus for promoting HDR is an apparatus that increases discharge of factors associated with an inflammatory reaction, found in histiocytes such as Mφ to an hernia site by ultrasonic irradiation, so that new blood vessel growth and liquid factors associated with disintegration of tissues in the prolapsed IDs are increased and the shrinkage is increased, and is equipped with at least one ultrasonic transducer, an ultrasonic oscillator and mount means for mounting the ultrasonic transducer to a hernia site. The ultrasound thus emitted is a low-power ultrasonic wave with which hardly generates any heat, and specifically the ultrasound is set to have a frequency of 1.3 to 2MHz, a repetition frequency of 100 to 1, 000Hz, a burst width of 10 to 2000µs and power of 100mW/cm² (SATA) or less, preferably it is a low-power ultrasonic wave of 1 to 100mW/cm² (SATA). As an irradiation method, it is preferable that a low-power ultrasonic pulse is transmitted to an affected part for 20 minutes per day, irradiation being carried out continuously during that period. An ultrasonic output unit produced by Exogen company in U.S.A. (SAFHS2000J™) may be used as the apparatus, and the characteristic of the ultrasonic output may be set to 200µs burst width, 1.5MHz frequency, 1kHz repetition frequency and 30-mW/cm² power.

The construction of the ultrasonic oscillator comprises a non-invasive medical treatment apparatus for transmitting a signal from the ultrasonic oscillator equipped in the main body through a cable to an ultrasonic medical treatment head module containing an ultrasonic transducer, and emitting an ultrasonic pulse having the above characteristics from the outside of the body in the proximity to the affected part through the skin. The contact between the ultrasonic medical treatment head module and the skin is carried out through ultrasonic-transmissible gel, whereby irradiation can be carried out without attenuation.

### [Main Body]

The main body comprises a power source, a storage operating device and an input/output device.

### 1) Power source:

An unchargeable and high-capacity type lithium battery is used as the battery. No power source switch is provided, power is turned on by operating a front panel, and the power is automatically turned off when the medical treatment for 20 minutes is completed. Furthermore, in order to prevent ultrasonic output due to erroneous operation, no ultrasonic wave is output unless a start button is pushed twice. Power is supplied from the same battery to the ultrasonic medical treatment head module.

### 2) Storage Operating Device:

CPU (Central Processing Unit) is mounted on a print board, and it carries out not only management of the medical treatment time, but also the operation of the ultrasonic medical treatment head module, the monitoring of the mount state and the self-diagnosis. Furthermore, the storage operating device is designed so that medical treatment records of patients are recorded in a back-up memory and can be taken out as occasion demands.

### 3) Input/Output Device

In addition to an operating switch, a liquid crystal display panel and a buzzer, the control of the medical treatment head module and the power supply are carried out through a connection cable. A signal of 200µs ultrasonic pulse burst width and 1KHz repetition frequency is transmitted as a control signal of the ultrasonic medical treatment head module. A trouble signal is sent back from the ultrasonic medical treatment head module.

### [Ultrasonic Medical Treatment Head Module]

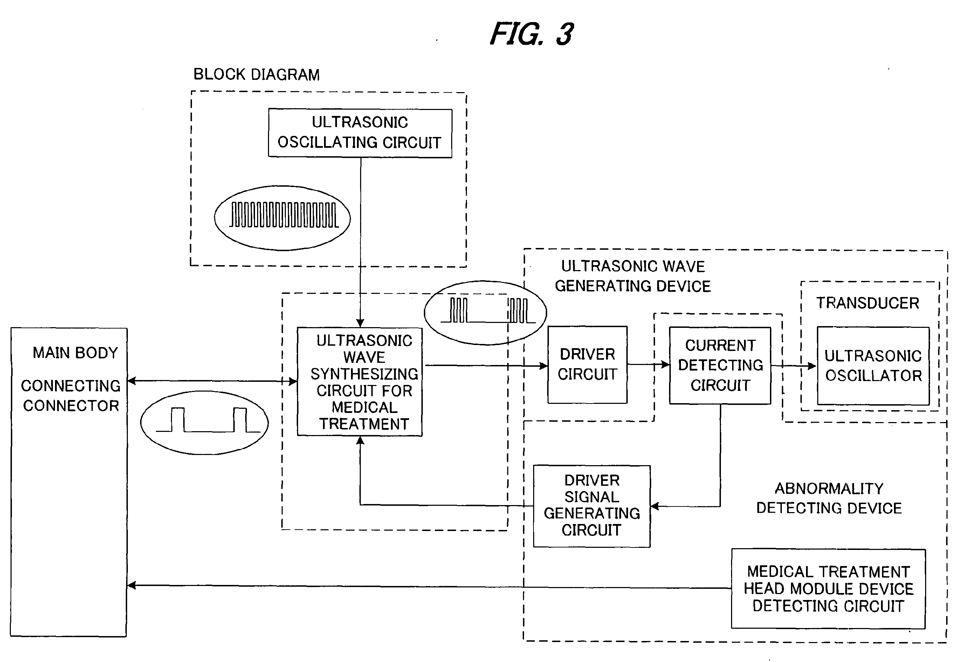

As shown in Fig. 3, the ultrasonic medical treatment head module comprises an oscillating device, an ultrasonic wave form synthesizing device, an ultrasonic wave generating device and an abnormality detecting device. The connection cable to the main body is designed integrally.
1) Oscillation device: the oscillation device generates an ultrasonic signal of 1.5MHz.
2) ultrasonic wave form synthesizing device: combines a control signal from the main body with an ultrasonic signal of 1.5MHz, and generates an electrical signal for an ultrasonic wave for medical treatment having a waveform with the signal characteristics: ultrasonic frequency 1.5MHz, ultrasonic power 30mW/cm², burst width 200µs, and repetition frequency 1kHz.
3) ultrasonic generating device: the piezoelectric ceramic has the characteristic that a minute distortion occurs in it when a voltage is applied thereto. By using this characteristic, an electrical signal generated in the ultrasonic wave synthesizing device is applied to a piezoelectric ceramic in the transducer, and vibration (ultrasonic wave) in the longitudinal direction is generated on the surface of the transducer and thereby used for medical treatment. An ultrasonic wave absorber is attached to the back surface of the piezoelectric ceramic to prevent leakage of ultrasonic wave.
4) abnormality detecting device: a medical treatment head detects that it is fixed to a fixing member and detects insufficiency of ultrasonic wave transmitting gel, and transmits the detection result to the main body, which warns patients with a liquid crystal display and an alarm. The detection of fixing failure between the medical treatment head and the fixing member is carried out by judging whether there is physical contact, on the basis of the presence or absence of conductivity. The insufficiency of gel is checked by detecting that driving current is increased to be higher than the normal value due to variation of the resonance property of the ultrasonic vibrator caused by lack of gel.

### [Mount means]

With respect to HD, when the NP and AF portion of the ID of vertebra or lumbar vertebra are protruded, for example in the fourth lumbar disc or fifth lumbar disc, the ischiadic nerve is compressed, so that back pain such as ischiaglia is caused. The mount means is mounted on the back close to the cervical vertebra, vertebra or lumbar vertebra which is the area of HD, being equipped with a housing portion for fixing the ultrasonic medical treatment head module containing.the ultrasonic transducer to the skin of the back. The mount means is fixed by a belt or the like.

### Examples

An effect of promoting the HDR when the apparatus for promoting the HDR according to the present invention will be described in embodiments. The effect of ultrasonic irradiation on the HDR has been confirmed by using rats.

### [Ultrasonic irradiation device for experiments]

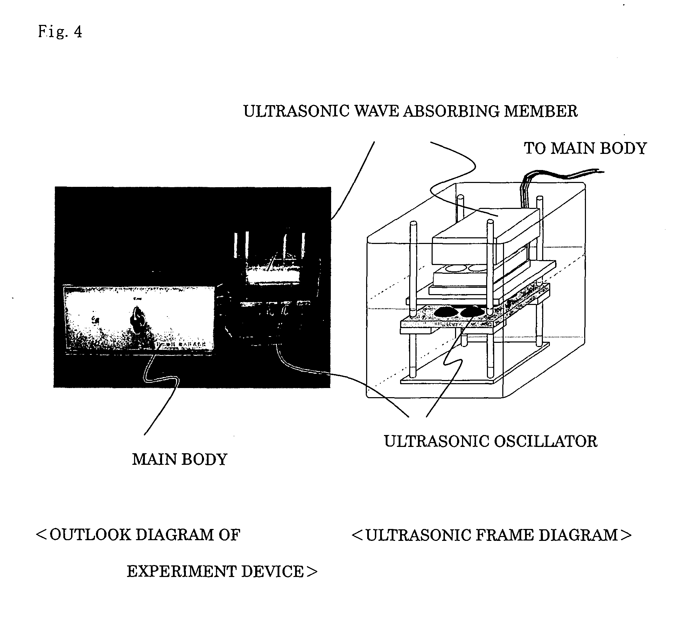

An ultrasonic irradiation device for animal experiments is used by partially modifying the construction of the apparatus for promoting HDR. First, an oscillating device and an ultrasonic wave form synthesizing device are placed in the main body, and the apparatus is modified so that six ultrasonic wave generating devices can be controlled at the same time, and the abnormality detecting circuit is eliminated. Furthermore, as shown in Fig. 4, a frame for fixing the six ultrasonic wave generating devices in a water tank and an ultrasonic wave absorbing member for preventing reflection of ultrasonic waves are added. When ultrasonic waves are emitted, the ultrasonic wave absorbing member is put on the upper surface of a culture dish to prevent the reflection of the ultrasonic waves between the culture solution and the air layer. The ultrasonic output condition is set to the same as the apparatus of Fig. 3, that is, the ultrasonic wave is set to have the characteristics: ultrasonic frequency 1.5MHz, ultrasonic wave power 30mW/cm²(SATA), burst width 200µs, and repetition frequency 1KHz.

### [Condition for Creating Model for Natural shrinkage of Hernia of Intervertebral disc]

Experiments were carried out by using rats according to a model for HDR which has devised by Namiro, et. al. (see The Journal of Clinical Investigation, Vol. 105, 2000). The experiments were also carried out on a model for HDR in mice, but it was difficult to detect notable HDR by the ultrasonic irradiation because the IDs of the mice were very small. Therefore, a model for a rat, whose IDs are larger than that of the mouse(about 10 times), has been developed. Specifically, 0.1cc/g of 4.05% thioglycolate medium (produced by Difco company, BREWR TIOGLYCOLATE MEDIUM) was injected into the abdominal cavity of a rat (male, weight of 150 to 250g), and Mφs were collected after three to four days. A tail IDs of the rat (male, weight of 150 to 200g) were collected, and co-cultivated with the Mφ (cell density: 12,000,000/well) after the wet weights of the ID was measured. After four days of culture, the wet weights were measured again. Using the thus-constructed model for the HDR, the ID and the Mφs were irradiated everyday with low-power ultrasonic waves, using the ultrasonic wave irradiating experimental device for the experiments, and the effect of the ultrasonic irradiation was evaluated through the analysis of the wet weight variation of the ID, the measurement of the protein concentration in the culture liquid and the histologic verification based on immunostaining.

As an experiment group, the following six groups and a control group were set, and the effect of the ultrasonic irradiation was confirmed.

### [Ultrasonic wave irradiation group]

Ultrasonic wave irradiated group 1: With the ultrasonic wave output unit generating ultrasonic waves, the co-cultivation system of the ID and Mφs was irradiated for 20 minutes per day, for all the days of the culture (18 examples) .

Ultrasonic wave irradiated group 2: With the ultrasonic wave output unit generating ultrasonic waves, the cultivation system of the ID was irradiated for 20 minutes per day, for all the days of the culture (5 examples).

Ultrasonic wave irradiated group 3: With the ultrasonic wave output unit generating ultrasonic waves, the cultivation system of Mφs was irradiated for 20 minutes per day, for all the days of the culture (5 examples).

### [Ultrasonic wave non-irradiated group]

Ultrasonic wave non-irradiated group 1: a ultrasonic wave output unit generating no ultrasonic waves was used for the co-culture system of the ID and Mφs for 20 minutes per day, for all the days of the culture (5 examples).

Ultrasonic wave non-irradiated group 2: a ultrasonic wave output unit generating no ultrasonic waves was used for the culture system of the ID for 20 minutes per day, for all the days of the culture (5 examples).

Ultrasonic wave non-irradiated group 3: a ultrasonic wave output unit generating no ultrasonic wave was used for the culture system of Mφs for 20 minutes per day, for all the days of the culture (5 examples).

### [Control Group]

Control group 1 : Normal cultivation was carried out under the state that the ID and Mφs coexist (5 examples).

Control group 2: Only the ID was normally cultivated (5 examples).

Control group 3: Only Mφs were normally cultivated (5 examples).

### [Estimating Method]

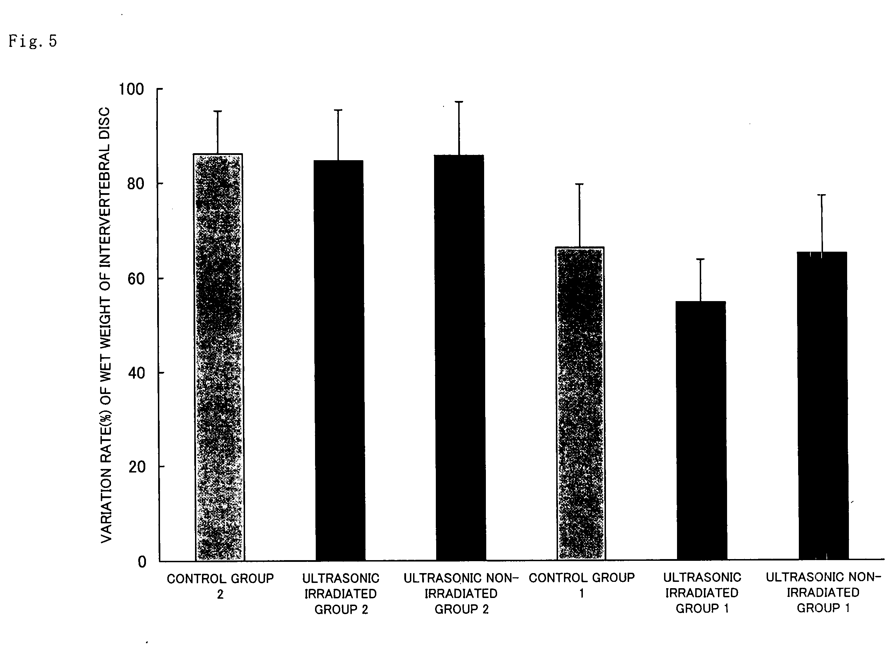

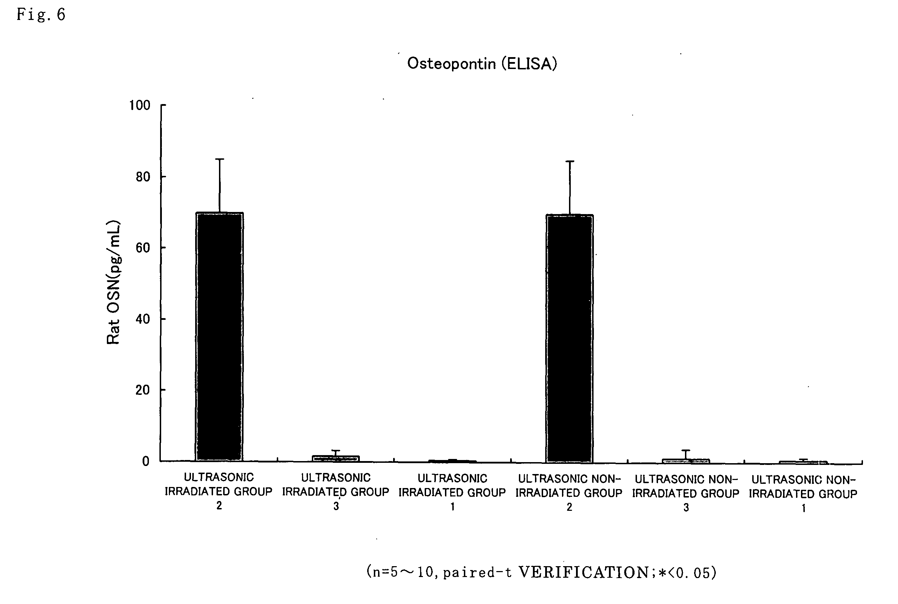

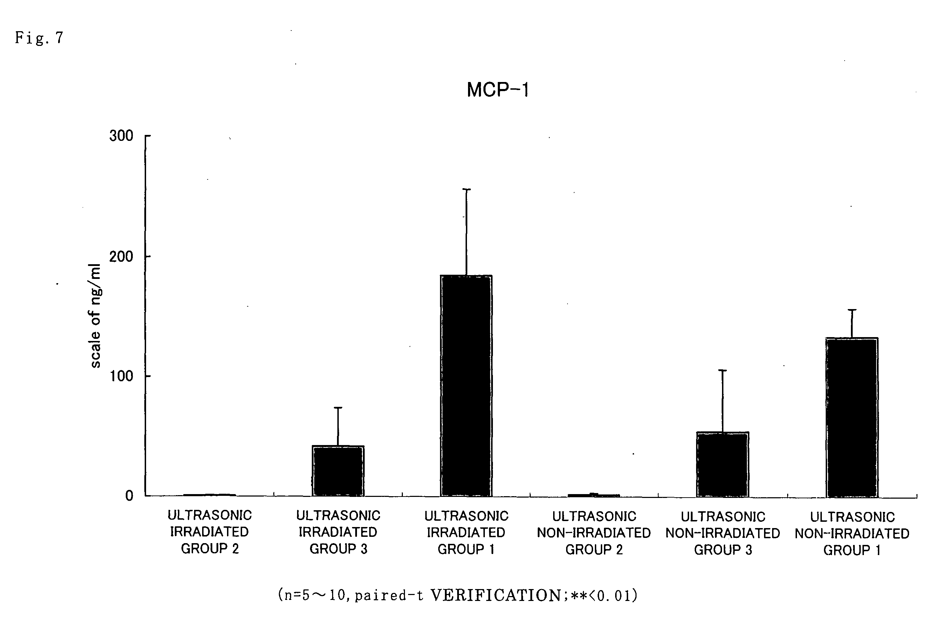

After the IDs were collected, only the IDs were cultivated for two hours (in order to allow the culture medium to infiltrate into the ID), and then the wet weights of the IDs were measured. Thereafter, the co-culture was carried out, and ultrasonic irradiation was carried out for four days. Thereafter, the wet weight of each ID was measured again, and the change in the wet weight was calculated (Fig. 5). Thereafter, the cell supernatant solution was collected, and the concentration of osteopontin was measured by the ELISA method. A kit produced by Immuno-Biological Laboratories Co. , Ltd. was used for the measurement (Fig. 6). Osteopontin contributes to infiltration of Mφs, and it is discharged from the chondrocytes in the ID. Likewise, the concentration of the cell supernatant solution of MCP-1 was measured by the ELISA method (Fig. 7). A kit produced by Immuno-Biological Laboratories Co. was used for the measurement. MCP-1 is a monocyte chemotactic factor, discharged by Mφs and associated with the inflammatory reaction.

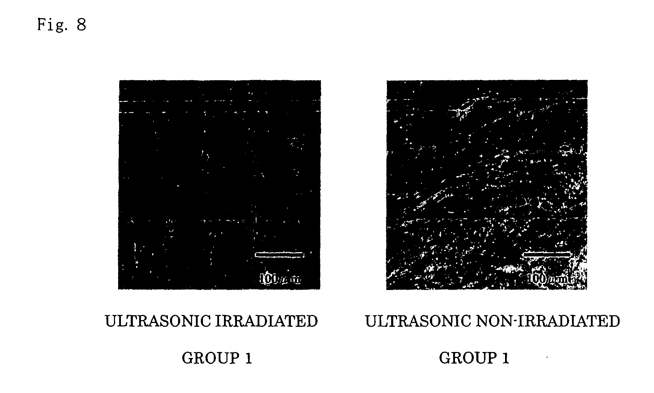

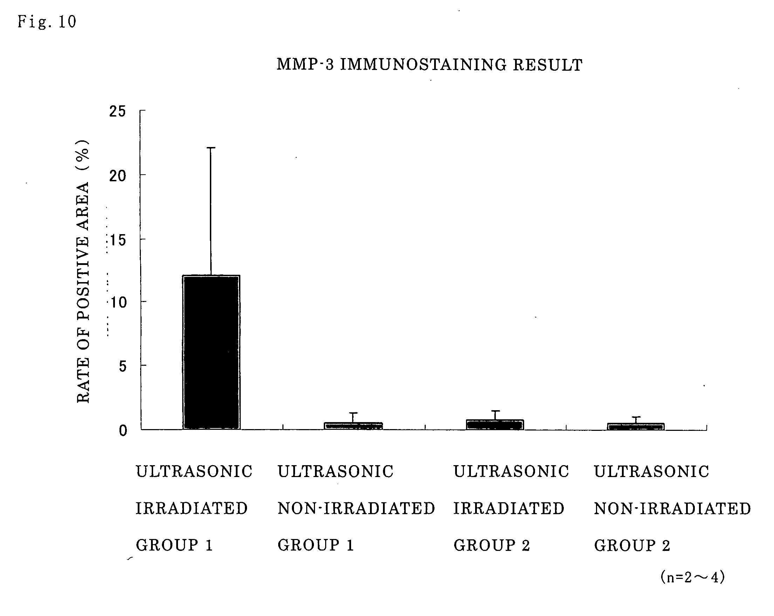

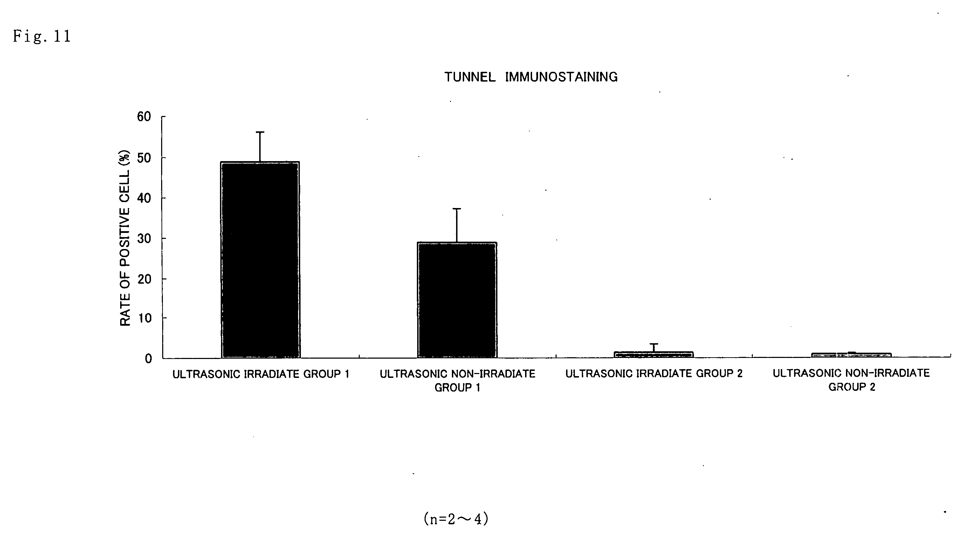

Furthermore, cut tissue pieces of ID were made, and the tissue pieces were subjected to haematoxylene/eosin (HE) staining, Safranin-0 staining (produced by Wako Pure Drug Industries, Ltd.), stromelysin 1 (MMP-3) staining (produced by Daiichi Fine Chemical Co., Ltd.), and apoptosis cell death staining (produced by Cosmo Bio Co., Ltd.), and the tissue structure of the ID, etc. was evaluated under an optical microscope (Figs. 8 to 11, table 1).

The methods of Safranin-0 staining is specifically reacted the cartilage matrix. Furthermore, MMP-3 contributes to the decomposition of the core protein and the extracellular matrix components of proteoglycan.

### [Results]

In the ultrasonic wave irradiated group 1 (co-culture of ID and Mφs + stimulation of ultrasonic wave), the wet weights of the ID were reduced by about 45.2%, and it exhibited the most remarkable reduction as compared with the other groups (Fig. 5). Furthermore, the ultrasonic wave irradiated group 1 exhibited the smallest expression amount of Osteopontin, and conversely the greatest expression amount of MCP-1. This suggests that infiltration of Mφs into the ID is more efficiently carried out in this group than in the other groups (Fig. 6, Fig. 7).

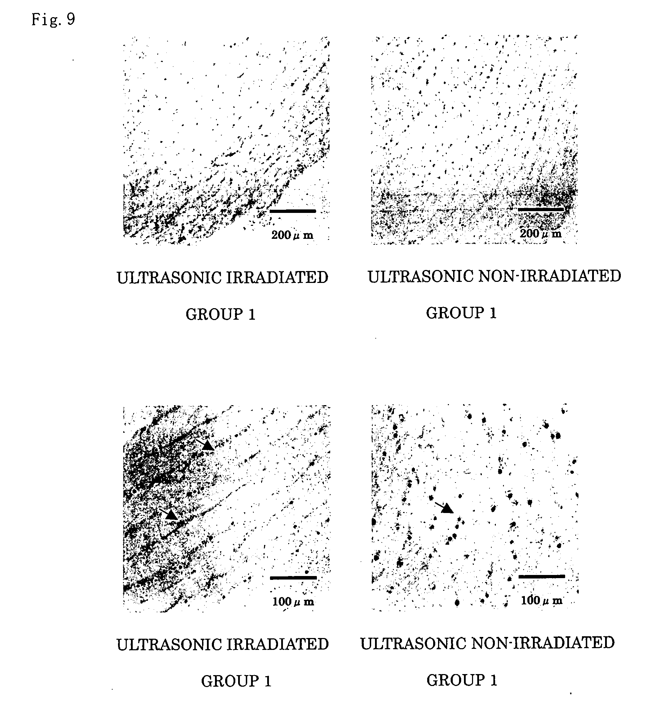

As a result of HE staining and Safranin-0 staining, condensation of nucleuses of cartilage cells constituting ID and irregularity of AF were confirmed in the ultrasonic wave irradiated group 1 (Fig. 8, table 1). This may be because much MMP-3 develops in the extracellular matrix, so that apoptosis occurred (Figs. 9 to 11).

**Table 1 Evaluation based on HE dyed image**

| | Ultrasonic wave irradiated group 1 | Ultrasonic wave non-irradiated group 1 | Ultrasonic wave irradiated group 2 | Ultrasonic wave non-irradiated group 2 |
|---|---|---|---|---|
| Reduction of cartilage cell | ++ | ++ | +- | +- |
| Aggregation of nucleus of cartilage cell | ++ | ++ | +- | +- |
| Acidophilic variation of cartilage cellular cytoplasm | + | + | - | - |
| Hypertrophy of cartilage cell | - | - | ++ | ++ |
| Irregularity of annulus fibrosus | + | - | - | - |
| Reduction of extracellular matrix | + | - | - | - |

| | | | | |
|---|---|---|---|---|
| -: no variation, +-: scanty variation, +: slight degree of variation, ++: middle degree of variation | | | | |

Summarizing the foregoing results, it has been found that in the ultrasonic wave irradiated group 1,
1) the wet weights of the ID are reduced the most, 2) the expression amount of MPC-1 is increased the most, 3) the expression amount of Osteopontin is reduced the most, 4) MMP-3 is expressed in the extracellular matrix the most, 5) irregularity of AF and reduction of extracellular matrix occur, 6) aggregation and shrinkage of nucleuses in cells occur, and 7) the number of cartilage cells is reduced due to apoptosis, so that the resorption is promoted.

With the HDR model system, it has been suggested that the liquid factors such as MPC-1 from Mφs are increased by the ultrasonic stimulation, and the cells constituting the IDs react to the above elements by increasing expression of MMP-3, so that the cells constituting of the IDs die due to increase of extracellular matrix.

### Effect of the Invention

According to the present invention, irradiation with ultrasonic waves promotes the disintegration of tissue of hernia. By using the apparatus of the present invention, the disc herniation can be non-invasively cured by ultrasonic irradiation for a short period of time, such 20 minutes per day, while staying at home.

## Claims

1. An apparatus for promoting the natural herniated disc resorption (HDR) by irradiation with ultrasonic waves, comprising: at least one ultrasonic transducer; an ultrasonic oscillator; and mount means for mounting the ultrasonic transducer to a site of herniated disc (HD).

2. The apparatus for promoting the natural HDR according to claim 1, wherein the ultrasonic transducer comprises means for emitting ultrasonic waves having a frequency of 1.3 to 2MHz, a repetition frequency of 100 to 1,000Hz, a burst width of 10 to 2000µs, and power of 1 to 100mW/cm² (SATA: Spatial Average-Temporal Average).

3. The apparatus for promoting the natural HDR according to claim 2, wherein the ultrasonic waves have frequency 1. 5MHz, repetition frequency 1kHz, burst width 200µs and power 30mW/cm².

4. A method of promoting the natural HDR, wherein ultrasonic waves are emitted from the surface of skin to the site of HD.

5. The method of promoting the natural HDR according to claim 4, wherein the ultrasonic waves have a frequency of 1.3 to 2MHz, a repetition frequency of 100 to 1,000Hz, a burst width of 10 to 2000µs and power of 1 to 100mw/cm² (SATA: Spatial Average-Temporal Average).

6. The method of promoting the natural HDR according to claim 5, wherein the ultrasonic waves have frequency 1.5MHz, repetition frequency 1kHz, burst width 200µs and power 30mW/cm².

7. A medical treatment method for HD, wherein ultrasonic waves are irradiated from the surface of skin to a site of HD.

8. The medical treatment method for HD according to claim 7, wherein the ultrasonic waves have a frequency of 1.3 to 2MHz, a repetition frequency of 100 to 1,000Hz, a burst width of 10 to 2000µs, and power of 1 to 100mW/cm² (SATA: Spatial Average-Temporal Average).

9. The medical treatment method for HD according to claim 8, wherein the ultrasonic waves are set to have frequency 1.5MHz, repetition frequency 1kHz, burst width 200µs and power 30mW/cm².
